(19) Europäisches Patentamt
European Patent Office
Office européen des brevets

(11) EP 0 747 043 B1

(12) # FASCICULE DE BREVET EUROPEEN

(45) Date de publication et mention de la délivrance du brevet:
**09.04.1997 Bulletin 1997/15**

(51) Int. Cl.$^6$: **A61K 7/48**

(21) Numéro de dépôt: **96400478.2**

(22) Date de dépôt: **06.03.1996**

(54) **Utilisation de dérivés d'acide salicyclique pour la depigmentation de la peau**

Verwendung von Salicylsäure-Derivativen zur Hautbleichung

Use of salicylic acid derivatives for skin whitening

(84) Etats contractants désignés:
**DE ES FR GB IT**

(30) Priorité: **07.04.1995 FR 9504189**

(43) Date de publication de la demande:
**11.12.1996 Bulletin 1996/50**

(73) Titulaire: **L'OREAL**
**75008 Paris (FR)**

(72) Inventeurs:
- **Causse, Catherine**
  **F-75011 Paris (FR)**
- **Deprez, Sabine**
  **F-94320 Thiais (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
**EP-A- 0 378 936**
**EP-A- 0 570 230**
**EP-A- 0 662 318**
**EP-A- 0 680 748**
**WO-A-91/05543**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.14.2/3.4

## Description

L'invention se rapporte à l'utilisation de dérivés d'acide salicylique dans ou pour la préparation d'une composition cosmétique ou dermatologique par application topique sur la peau du visage et/ou du corps, dans le but de blanchir la peau ou de traiter les taches pigmentaires sans entraîner de desquamation de la peau.

A différentes périodes de leur vie, certaines personnes voient apparaître sur la peau et plus spécialement sur les mains, des taches plus foncées et/ou plus colorées, conférant à la peau une hétérogénéité. Ces taches sont dues à une concentration importante de mélanine dans les kératinocytes situés à la surface de la peau. En effet, les mélanocytes situés dans la partie profonde de l'épiderme produisent de la mélanine et délivrent cette mélanine aux kératinocytes environnants, qui vont ensuite remonter à la surface de l'épiderme, chargés de mélanine.

Le mécanisme de formation de la pigmentation de la peau, c'est-à-dire de la formation de la mélanine est particulièrement complexe et fait intervenir schématiquement les principales étapes suivantes :

Tyrosine ---> Dopa ---> Dopaquinone ---> Dopachrome ---> Mélanine

La tyrosinase est l'enzyme essentielle intervenant dans cette suite de réactions. Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de la toxicité qu'ils entraînent, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi l'hydroquinone, dont l'emploi est d'ailleurs limité légalement à une concentration de 2 %, est un composé particulièrement irritant et cytotoxique pour le mélanocyte, dont le remplacement, total ou partiel a été envisagé par de nombreux auteurs.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo. Pour ces dernières hyperpigmentations, à défaut de pouvoir repigmenter la peau lésée, on achève de dépigmenter les zones de peau normale résiduelle pour donner à l'ensemble de la peau une teinte blanche homogène.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique. Par ailleurs, le document US5262153 enseigne d'utiliser l'acide lactique et ses dérivés.

La demanderesse a trouvé de manière inattendue que certains dérivés d'acide salicylique présentaient la propriété d'inhiber l'activation de la tyrosinase, et donc son activité, et d'agir ainsi sur la pigmentation et les taches de la peau sans toxicité.

Certes, il est connu du document EP-A-378936 d'utiliser ces dérivés d'acide salicylique comme agents kératolytiques pour prévenir le vieillissement de la peau et, entre autres, pour traiter les taches pigmentées d'origine sénile. Ces agents kératolytiques sont des exfoliants ou desquamants et réduisent les taches pigmentaires en éliminant les cellules mortes en surface de la peau et notamment celles de ces taches. Ce document n'enseigne nullement que ces dérivés d'acide salicylique peuvent inhiber l'activité de la tyrosinase en ayant pour cible les mélanocytes, et permettre ainsi une réduction de la production de mélanine.

Or, la demanderesse a constaté de manière surprenante que ces composés, quand on les utilisait à faible concentration, avaient un effet d'inhibition de l'activité de la tyrosinase et dépigmentaient la peau sans la desquamer, en agissant sur la synthèse de la mélanine et non en éliminant les cellules mortes. Ces deux processus d'action, synthèse de la mélanine et desquamation, sont bien différents et même antinomiques.

En effet, quand un composé desquame la peau, il crée une inflammation superficielle de la peau, entraînant la stimulation de la mélanine. Par conséquent, la desquamation conduit à une effacement des taches superficielles de la peau et non à une inhibition de la production de mélanine. C'est ainsi que les composés décrits dans le document EP-A-378936, utilisés à des concentrations supérieures à 3 %, effacent les taches de la peau par desquamation. Rien ne laissait supposer qu'à faible concentration, ces mêmes composés pouvaient ralentir ou limiter la synthèse de la mélanine et avoir alors au contraire un effet anti-inflammatoire. Selon les concentrations utilisées, ces composés ont de manière surprenante des effets contraires sur la peau.

En outre, le document EP-A-570230 décrit l'utilisation de dérivés alkylés et alcoxylés de l'acide salicylique à fortes concentrations ou en association avec d'autres dépigmentants, pour traiter la dyschromie. Cependant, il n'enseigne pas que d'autres dérivés d'acide salicylique utilisés seuls puissent avoir une telle propriété à des concentrations beaucoup plus faibles. En outre, des composés même de formule proche peuvent avoir des propriétés bien différentes.

Aussi, la présente invention a pour objet l'utilisation, comme seul inhibiteur de la tyrosinase, dans et/ou pour la préparation d'une composition cosmétique et/ou dermatologique, d'un dérivé d'acide salicylique de formule (I) ou d'un sel d'un tel dérivé :

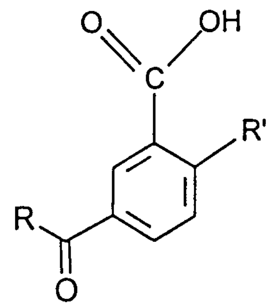

(I)

dans laquelle :

R représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;

R' représente un groupement hydroxyle ou une fonction ester de formule :

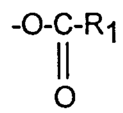

où $R_1$ est un groupement aliphatique saturé ou insaturé ayant de 1 à 18 atomes de carbone.

De préférence, le radical R comporte au moins 4 atomes de carbone. Il est par exemple formé d'un radical alkyle linéaire saturé ayant de 4 à 11 atomes de carbone.

L'application d'une composition contenant un dérivé d'acide salicylique selon l'invention permet d'obtenir une nette diminution voire une disparition complète de la formation de taches par inhibition de la tyrosinase et donc permet d'obtenir une inhibition de la synthèse de la mélanine.

Aussi, la présente invention a encore pour objet l'utilisation d'une quantité efficace d'un seul inhibiteur pour inhiber la synthèse de la mélanine, dans et/ou pour la préparation d'une composition cosmétique et/ou dermatologique, le dit inhibiteur étant un dérivé d'acide salicylique de formule (I) ou d'un sel d'un tel dérivé :

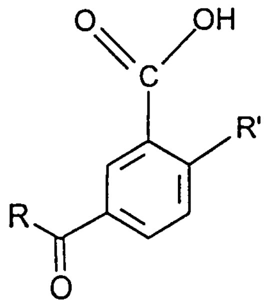

(I)

dans laquelle :

R représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;

R' représente un groupement hydroxyle ou une fonction ester de formule :

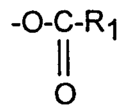

où $R_1$ est un groupement aliphatique saturé ou insaturé ayant de 1 à 18 atomes de carbone.

Ces composés à faible concentration présentent l'avantage de dépigmenter et blanchir la peau sans la desquamer et donc sans l'irriter, ce qui est tout à fait surprenant pour ce type de composés.

Aussi, l'invention a encore pour objet l'utilisation d'une quantité efficace d'un composé pour blanchir la peau sans la desquamer, dans et/ou pour la préparation d'une composition cosmétique et/ou dermatologique, le dit composé étant un dérivé d'acide salicylique de formule (I) ou d'un sel d'un tel dérivé :

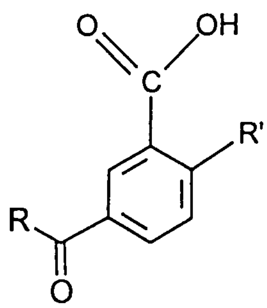

(I)

dans laquelle :

R représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;

R' représente un groupement hydroxyle ou une fonction ester de formule :

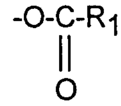

où $R_1$ est un groupement aliphatique saturé ou insaturé ayant de 1 à 18 atomes de carbone.

De façon avantageuse, le dérivé de l'acide salicylique est choisi parmi les acides n-octanoyl-5-salicylique, n-décanoyl-5-salicylique, n-dodécanoyl-5-salicylique.

Il peut s'agir également des sels de ces acides, et en particulier des sels obtenus par salification avec une base.

Comme base susceptible de salifier les dérivés de l'acide salicylique selon l'invention, on peut citer les bases minérales comme les hydroxydes de métaux alcalins (hydroxydes de sodium et de potassium) ou les hydroxydes d'ammonium ou mieux encore les bases organiques.

De préférence, on utilise des bases amphotères pour la salification des dérivés de l'acide salicylique, c'est-à-dire des bases ayant à la fois des groupements fonctionnels anioniques et cationiques.

Les bases amphotères peuvent être des amines organiques primaires, secondaires, tertiaires ou cycliques et plus spécialement des acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine. Ces bases sont utilisées en quantités suffisantes pour amener le pH de l'émulsion entre 5 et 7 et donc proche de celui de la peau. Il s'ensuit une grande compatibilité de l'émulsion de l'invention vis-à-vis de la peau.

Les dérivés d'acide salicylique ou leurs sels sont utilisés, selon la présente invention, de préférence en quantité allant de 0,01 à 2 % en poids et encore plus préférentiellement de 0,2 à 0,5 %.

Les compositions contenant les dérivés d'acide salicylique selon l'invention peuvent présenter toutes les formes galéniques normalement utilisées pour une application topique, par exemple sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

De façon connue, les compositions cosmétiques ou dermatologiques de l'invention peuvent contenir des adjuvants habituels dans le domaine cosmétique ou dermatologique, tels que les émulsionnants, les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans les domaines cosmétique et/ou dermatologique, et par exemple de 0,01 % à 20 % du poids total de la composition. Ces adjuvants, selon leur nature, peuvent être introduits dans la phase grasse, dans la phase aqueuse et/ou dans les vésicules lipidiques.

Comme émulsionnants, on peut utiliser des émulsionnants eau-dans huile (E/H) ou huile-dans-eau (H/E) selon l'émulsion finale souhaitée.

Comme émulsionnants, on peut citer par exemple le stéarate de PEG-20, le stéarate de PEG-100, le Polysorbate 60 (Tween 60 vendu par la société ICI, le stéarate de sorbitan (Span 60 vendu par la société ICI) et le PPG-3 myristyl éther.

Le taux d'émulsionnant peut aller de 0,1 % à 15 % en poids, et de préférence de 0,5 % à 5 % en poids, par rapport au poids total de la composition.

On peut ajouter à la composition selon l'invention des coémulsionnants, par exemple en une quantité allant de 0,05 % à 10 % en poids par rapport au poids total de la composition. Comme coémulsionnant, on peut citer le stéarate de glycérol.

Dans les dispersions de vésicules lipidiques, l'émulsionnant peut être constitué par des vésicules de lipides ioniques et/ou non ioniques.

Comme huiles utilisables dans l'invention, on peut citer les huiles minérales, les huiles végétales (huile de tournesol, huile d'amande d'abricot, huile de karité), les huiles de synthèse, les huiles siliconées (cyclométhicone) et les huiles fluorées (perfluoropolyéthers). On peut ajouter à ces huiles des alcools gras (alcool stéarylique), des acides gras (acide stéarique) et des cires.

Comme gélifiants hydrophiles, on peut citer les polymères carboxyvinyliques, les polyacrylates ou polyméthacrylates de glycéryle, les polyacrylamides, les gommes naturelles (xanthane) et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser par exemple les protéines ou les hydrolysats de protéine, les acides aminés, les polyols, en particulier la glycérine ou le sorbitol, l'urée, l'allantoïne, les sucres et leurs dérivés, l'acide glycyrrhétinique.

Comme actifs lipophiles, on peut citer le tocophérol (vitamine E) et ses dérivés, les acides gras essentiels, les céramides, les huiles essentielles.

On peut aussi utiliser dans ces compositions des filtres UV à propriété lipophile ou hydrophile, les oxydes de titane et de zinc, éventuellement sous forme de nano-oxydes, notamment en vue d'obtenir des compositions assurant également une bonne protection contre les UV.

Ces compositions constituent notamment des crèmes de protection, de traitement ou de soin pour le visage, pour les mains ou pour le corps, des laits corporels de protection ou de soin, des lotions, gels ou mousses pour le soin ou le traitement de la peau, des lotions de nettoyage ou de désinfection, des compositions pour le bain, des fonds de teint et des crèmes teintées. Dans ces derniers cas, la composition contient des pigments.

**Test:**

Un test a mis en évidence l'activité de l'acide n-octanoyl-5 salicylique comme inhibiteur de la tyrosinase par évaluation de son effet sur l'activité dopa-oxydase de la tyrosinase des mélanocytes humains.

Des mélanocytes humains ont été préparés et mis en culture, puis introduits dans des milieux comportant différentes concentrations d'acide n-octanoyl-5 salicylique (0,5 μM, 1 μM, 2 μM et 5 μM).

Après trois jours, les mélanocytes ont été « trypsinisés » dans un mélange de trypsine / EDTA 0,05 % / 0,02 % dans un tampon phosphate (50 mM à pH 6,8), lavés à trois reprises dans le tampon phosphate contenant 1 % (w/v) de Triton x-100 et soumis à des ultrasons. Après centrifugation, des échantillons ont été prélevés et mélangés avec de la L-DOPA 5 mM dans du tampon phosphate. L'activité en tyrosinase a été évaluée par spectrophotométrie en mesurant l'augmentation de l'absorbance à 475 nm due à la formation de dopachrome à partir de DOPA 5 mM. La lecture de l'absorbance a été effectuée sur un lecteur de microplaques à 25°C pendant 60 mn.

L'activité de la tyrosinase cellulaire est mesurée par rapport à une gamme étalon réalisée avec la tyrosinase commerciale.

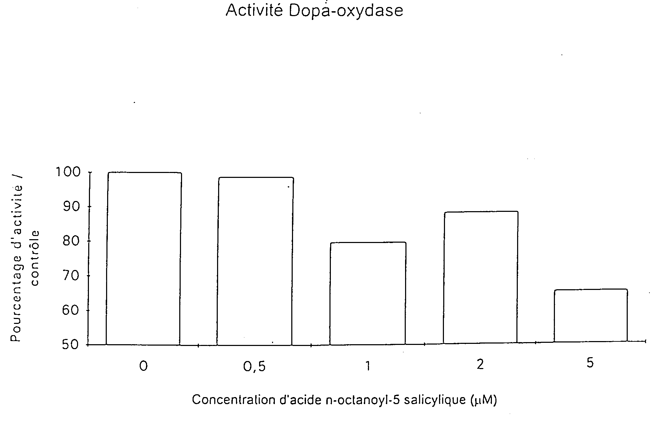

Les résultats sont présentés sur la figure 1 qui donne en abscisse les concentrations d'acide n-octanoyl-5 salicylique, exprimées en μM, en fonction du pourcentage d'activité par rapport à l'activité de départ en ordonnée. Ces histogrammes montrent que l'activité dopa-oxydase de la tyrosinase des mélanocytes traités par l'acide n-octanoyl-5 salicylique diminue de façon significative, plus particulièrement prononcée à la concentration de 5 μM.

Ces résultats montrent donc que l'acide n-octanoyl-5 salicylique module la mélanogénèse cellulaire en inhibant l'activité dopa-oxydase de la tyrosinase, c'est à dire la transformation de la L-DOPA en dopaquinone.

Les exemples suivants illustrent l'invention. Dans ces exemples, les proportions indiquées sont des pourcentages en poids.

**Exemple 1 : Emulsion H/E**

| | |
|---|---|
| Acide n-octanoyl-5 salicylique | 1 % |
| Octyldodécanol | 5 % |
| Huile de tournesol | 11 % |
| EDTA | 0,05 % |
| Hydroxyde de sodium | 0,02 % |
| Gomme de xanthane | 0,2 % |
| Polyacrylamide/Isoparaffine/Laureth-7 (Sepigel 305 vendu par la société Seppic) | 0,9 % |
| Cyclométhicone | 5 % |
| Glycérine | 4 % |
| Polyacrylate de glycéryle à 2 % dans un mélange eau/glycérine (Lubrajel vendu par la société Guardian) | 5 % |
| Stéarate de glycérol | 0,6 % |
| Stéarate de PEG-100 | 0,6 % |
| Stéarate de PEG-20 | 1,2 % |
| Acide stéarique | 0,6 % |
| Alcool stéarylique | 1 % |
| Conservateurs | 0,3 % |
| Eau | qsp 100 % |

On obtient une crème fluide de couleur blanche à propriété dépigmentante.

Un test sur un panel de 20 femmes a montré que cette crème est facile à appliquer, pénètre bien et a une texture agréable.

Par ailleurs, cette crème a fait l'objet d'un test cosmétoclinique sur 22 femmes âgées de 39 à 65 ans, présentant des taches sur les mains et le visage.

La crème a été appliquée sur le visage et sur la face dorsale d'une main pendant 4 semaines (28 jours) à raison d'une application chaque soir.

Les femmes ont conservé leurs habitudes d'hygiène et de maquillage.

L'atténuation des taches a été évaluée par chromamètre avant le début du traitement ($J_0$) et après le traitement ($J_{29}$) : la coloration d'une tache repérée de la main a été mesurée à l'aide du chromamètre CR200 (Minolta). Parallèlement, des mesures ont été effectuées sur le site adjacent de peau saine servant de zone témoin. Les mesures ont été faites à trois reprises dans le système L* et b*, définissant l'espace couleur de la peau :

- L* définissant la luminosité
- b* définissant la couleur de la peau dans l'axe vert-bleu.

A partir de ces mesures, on a calculé l'angle ITA (Individual Typological Angle) en dégré (°), permettant de classer par catégories les couleurs de peau, selon la formule :

$$\text{Angle ITA}° = [\text{Arc tangente } ((L^*-50)/b^*)] \times 180/3{,}1416.$$

La significativité des écarts entre les valeurs d'angle ITA° prises sur zone témoin et sur zone pigmentée à $J_0$ et à $J_{29}$ a été évaluée par le test de Student sur séries appariées, donnant la valeur de « t ».

L'effet inhibiteur de la tyrosinase de la crème a été apprécié par sa capacité à estomper la couleur de la tache et à

lui donner une couleur comparable à celle de la peau saine adjacente.

| | $J_0$ | $J_{29}$ | $J_{29}$-$J_0$ | « t » | résultat | seuil de significativité |
|---|---|---|---|---|---|---|
| Tache | 21,20 | 26,65 | 5,45 | 4,459 | *** | 0,1 % |
| Témoin | 43,56 | 45,58 | 2,02 | 1,745 | ns | 10 % |
| Témoin -tache | 22,36 | 18,94 | 3,43 | 3,816 | ** | 1 % |
| Les résultats sont significatifs lorsque le seuil de significativité est inférieur à 5 %. Par ailleurs, le résultat très significatif est noté : « *** », le résultat significatif est noté : « ** » et le résultat non significatif est noté « ns ». | | | | | | |

Ce tableau montre que les valeurs $J_{29}$ - $J_0$ sont très significatives et significatives pour la tache et la différence (témoin - tache) et non significative pour le témoin. Ces résultats mettent en évidence que l'application de la crème a bien estompé les taches et diminué l'écart de coloration entre la zone témoin et la zone tachée.

**Exemple 2 : Emulsion H/E**

| | |
|---|---|
| Acide n-octanoyl-5 salicylique | 0,5 % |
| Huile d'amande d'abricot | 10 % |
| Huile de karité | 7 % |
| PPG-3 myristyl éther | 5 % |
| Polysorbate 60 (Tween 60) | 2,5 % |
| Stéarate de Sorbitan (Span 60) | 2,5 % |
| Conservateurs | 0,2 % |
| Cyclométhicone | 4 % |
| Gomme de xanthane | 0,2 % |
| Polymère carboxyvinylique | 0,5 % |
| Triéthanolamine (neutralisant) | 0,5 % |
| Glycérine | 5 % |
| eau | qsp 100 % |

On obtient une bonne crème de jour dépigmentante.

**Exemple 3 : Emulsion H/E**

On obtient une composition ayant des propriétés dépigmentantes et filtrantes en ajoutant 2,5 % en poids de nano-oxydes de titane dans la composition de l'exemple 1.

**Revendications**

1. Utilisation, comme seul inhibiteur de la tyrosinase, dans une composition cosmétique ou pour la préparation d'une composition dermatologique, d'un dérivé d'acide salicylique de formule (I) ou d'un sel d'un tel dérivé :

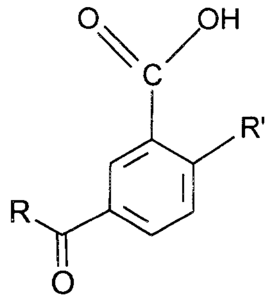

(I)

dans laquelle :

R représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule :

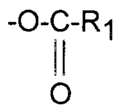

où $R_1$ est un groupement aliphatique saturé ou insaturé ayant de 1 à 18 atomes de carbone.

2. Utilisation d'une quantité efficace d'un seul inhibiteur pour inhiber la synthèse de la mélanine, dans une composition cosmétique ou pour la préparation d'une composition dermatologique, le dit inhibiteur étant un dérivé d'acide salicylique de formule (I) ou d'un sel d'un tel dérivé :

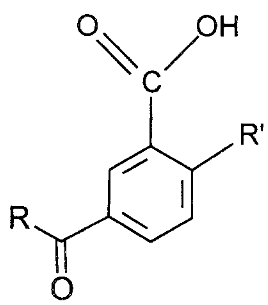

(I)

dans laquelle :

R représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;

R' représente un groupement hydroxyle ou une fonction ester de formule :

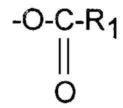

où $R_1$ est un groupement aliphatique saturé ou insaturé ayant de 1 à 18 atomes de carbone.

**3.** Utilisation d'une quantité efficace d'un composé pour blanchir la peau sans la desquamer, dans une composition cosmétique ou pour la préparation d'une composition dermatologique, le dit composé étant un dérivé d'acide salicylique de formule (I) ou d'un sel d'un tel dérivé :

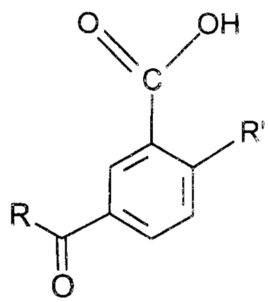

(I)

dans laquelle :

R représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;

R' représente un groupement hydroxyle ou une fonction ester de formule :

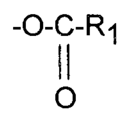

où $R_1$ est un groupement aliphatique saturé ou insaturé ayant de 1 à 18 atomes de carbone.

**4.** Utilisation selon l'une quelconque des revendications précédentes, caractérisée en ce que le radical R dans la formule (I) est un radical alkyle linéaire saturé ayant de 4 à 11 atomes de carbone.

**5.** Utilisation selon l'une quelconque des revendications précédentes, caractérisée en ce que le dérivé d'acide salicylique est choisi parmi les acides n-octanoyl-5-salicylique, n-décanoyl-5-salicylique, n-dodécanoyl-5-salicylique.

**6.** Utilisation selon l'une quelconque des revendications précédentes, caractérisée en ce que le dérivé d'acide salicylique est l'acide n-octanoyl-5 salicylique.

**7.** Utilisation selon l'une quelconque des revendications précédentes, caractérisée en ce que le dérivé d'acide salicylique est sous forme salifiée par une base minérale ou organique.

**8.** Utilisation selon l'une quelconque des revendications précédentes, caractérisée en ce que le dérivé d'acide salicylique ou son sel est présent en une concentration allant de 0,01 à 2 % en poids par rapport au poids total de la

composition.

9. Utilisation selon l'une quelconque des revendications précédentes, caractérisée en ce que la composition contient, en outre, un filtre UV.

10. Utilisation selon la revendication précédente, caractérisée en ce que le filtre UV est choisi parmi les oxydes de titane ou de zinc.

11. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux un gel huileux, un gel anhydre, un sérum, une dispersion de vésicules.

**Claims**

1. Use, as sole inhibitor of tyrosinase, in a cosmetic composition or for the preparation of a dermatological composition, of a salicylic acid derivative of formula (I) or of a salt of such a derivative:

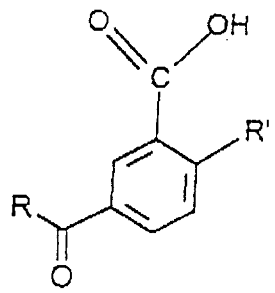

(I)

in which:

R represents a linear, branched or cyclized saturated aliphatic chain or an unsaturated chain carrying one or a number of double bonds, which may or may not be conjugated, these chains containing from 2 to 22 carbon atoms and being able to be substituted by at least one substituent chosen from halogen atoms, the trifluoromethyl group or hydroxyl groups in the free form or esterified by an acid having from 1 to 6 carbon atoms or else by a carboxyl functional group which is free or esterified by a lower alcohol having from 1 to 6 carbon atoms;
R' represents a hydroxyl group or an ester functional group of formula:

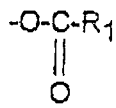

where $R_1$ is a saturated or unsaturated aliphatic group having from 1 to 18 carbon atoms.

2. Use of an effective amount of a sole inhibitor for inhibiting the synthesis of melanin, in a cosmetic composition or for the preparation of a dermatological composition, the said inhibitor being a salicylic acid derivative of formula (I) or a salt of such a derivative:

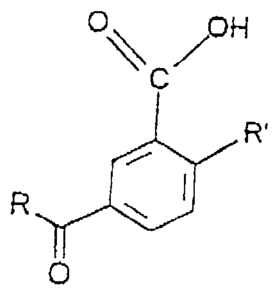

(I)

in which:

R represents a linear, branched or cyclized saturated aliphatic chain or an unsaturated chain carrying one or a number of double bonds, which may or may not be conjugated, these chains containing from 2 to 22 carbon atoms and being able to be substituted by at least one substituent chosen from halogen atoms, the trifluoromethyl group or hydroxyl groups in the free form or esterified by an acid having from 1 to 6 carbon atoms or else by a carboxyl functional group which is free or esterified by a lower alcohol having from 1 to 6 carbon atoms;
R' represents a hydroxyl group or an ester functional group of formula:

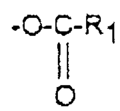

where $R_1$ is a saturated or unsaturated aliphatic group having from 1 to 18 carbon atoms.

**3.** Use of an effective amount of a compound for lightening the skin without desquamating it, in a cosmetic composition or for the preparation of a dermatological composition, the said compound being a salicylic acid derivative of formula (I) or of a salt of such a derivative:

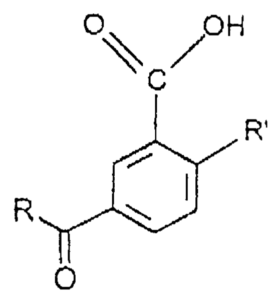

(I)

in which:

R represents a linear, branched or cyclized saturated aliphatic chain or an unsaturated chain carrying one or a number of double bonds, which may or may not be conjugated, these chains containing from 2 to 22 carbon atoms and being able to be substituted by at least one substituent chosen from halogen atoms, the trifluoromethyl group or hydroxyl groups in the free form or esterified by an acid having from 1 to 6 carbon atoms or else by a carboxyl functional group which is free or esterified by a lower alcohol having from 1 to 6 carbon atoms;
R' represents a hydroxyl group or an ester functional group of formula:

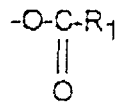

where $R_1$ is a saturated or unsaturated aliphatic group having from 1 to 18 carbon atoms.

4. Use according to any one of the preceding claims, characterized in that the R radical in the formula (I) is a saturated linear alkyl radical having from 4 to 11 carbon atoms.

5. Use according to any one of the preceding claims, characterized in that the salicylic acid derivative is chosen from 5-(n-octanoyl)salicylic, 5-(n-decanoyl)salicylic and 5-(n-dodecanoyl)salicylic acids.

6. Use according to any one of the preceding claims, characterized in that the salicylic acid derivative is 5-(n-octanoyl)salicylic acid.

7. Use according to any one of the preceding claims, characterized in that the salicylic acid derivative is in the form salified by an inorganic or organic base.

8. Use according to any one of the preceding claims, characterized in that the salicylic acid derivative or its salt is present in a concentration ranging from 0.01 to 2 % by weight with respect to the total weight of the composition.

9. Use according to any one of the preceding claims, characterized in that the composition additionally contains a UV screening agent.

10. Use according to the preceding claim, characterized in that the UV screening agent is chosen from titanium or zinc oxides.

11. Use according to any one of the preceding claims, characterized in that the composition is an aqueous, oily or aqueous/alcoholic solution, a water-in-oil emulsion, an oil-in-water emulsion, a microemulsion, an aqueous gel, an oily gel, an anhydrous gel, a serum or a vesicular dispersion.

**Patentansprüche**

1. Verwendung von Salicylsäurederivaten der Formel (I) oder Salzen dieser Derivate als einzigen Tyrosinaseinhibitor in einer kosmetischen Zusammensetzung oder zur Herstellung einer dermatologischen Zusammensetzung:

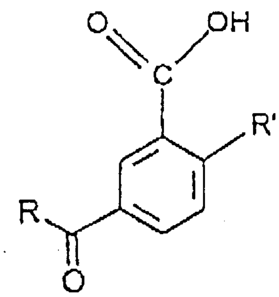

(I)

worin bedeuten:

R eine geradkettige, verzweigte oder cyclische gesättigte aliphatische Kette oder eine ungesättigte Kette mit einer oder mehreren Doppelbindungen, die gegebenenfalls konjugiert sind, wobei die Ketten 2 bis 22 Kohlenstoffatome aufweisen und mit mindestens einem Substituenten substituiert sein können, der unter den Halogenatomen, Trifluormethyl, einer Hydroxygruppe, die in freier Form vorliegen kann oder mit einer Säure mit 1 bis 6 Kohlenstoffatomen verestert ist, oder auch einer Carboxygruppe, die in freier Form vorliegt oder mit

einem niederen Alkohol mit 1 bis 6 Kohlenstoffatomen verestert ist, ausgewählt ist.
R' eine Hydroxygruppe oder eine Estergruppe der Formel:

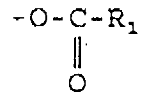

worin $R_1$ eine gesättigte oder ungesättigte aliphatische Gruppe mit 1 bis 18 Kohlenstoffatomen bedeutet.

**2.** Verwendung einer wirksamen Menge eines einzigen Inhibitors zur Hemmung der Melaninsynthese in einer kosmetischen Zusammensetzung oder zur Herstellung einer dermatologischen Zusammensetzung, wobei der Inhibitor ein Salicylsäurederivat der Formel (I) oder ein Salz eines dieser Derivate ist:

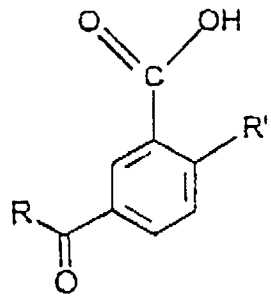

(I)

worin bedeuten:

R eine geradkettige, verzweigte oder cyclische gesättigte aliphatische Kette oder eine ungesättigte Kette mit einer oder mehreren Doppelbindungen, die gegebenenfalls konjugiert sind, wobei die Ketten 2 bis 22 Kohlenstoffatome aufweisen und mit mindestens einem Substituenten substituiert sein können, der unter den Halogenatomen, Trifluormethyl, einer Hydroxygruppe, die in freier Form vorliegen kann oder mit einer Säure mit 1 bis 6 Kohlenstoffatomen verestert ist, oder auch einer Carboxygruppe, die in freier Form vorliegt oder mit einem niederen Alkohol mit 1 bis 6 Kohlenstoffatomen verestert ist, ausgewählt ist.

R' eine Hydroxygruppe oder eine Estergruppe der Formel:

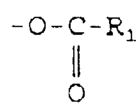

worin $R_1$ eine gesättigte oder ungesättigte aliphatische Gruppe mit 1 bis 18 Kohlenstoffatomen bedeutet.

**3.** Verwendung einer wirksamen Menge einer Verbindung zur Bleichung der Haut ohne sie abzuschuppen in einer kosmetischen Zusammensetzung oder zur Herstellung einer dermatologischen Zusammensetzung, wobei die Verbindung ein Salicylsäurederivat der Formel (I) oder ein Salz eines dieser Derivate ist:

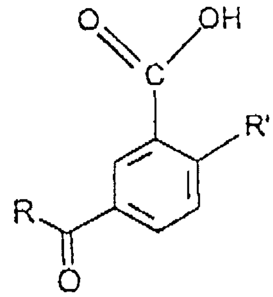 (I)

worin bedeuten:

R eine geradkettige, verzweigte oder cyclische gesättigte aliphatische Kette oder eine ungesättigte Kette mit einer oder mehreren Doppelbindungen, die gegebenenfalls konjugiert sind, wobei die Ketten 2 bis 22 Kohlenstoffatome aufweisen und mit mindestens einem Substituenten substituiert sein können, der unter den Halogenatomen, Trifluormethyl, einer Hydroxygruppe, die in freier Form vorliegen kann oder mit einer Säure mit 1 bis 6 Kohlenstoffatomen verestert ist, oder auch einer Carboxygruppe, die in freier Form vorliegt oder mit einem niederen Alkohol mit 1 bis 6 Kohlenstoffatomen verestert ist, ausgewählt ist.

R' eine Hydroxygruppe oder eine Estergruppe der Formel:

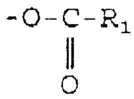

worin $R_1$ eine gesättigte oder ungesättigte aliphatische Gruppe mit 1 bis 18 Kohlenstoffatomen bedeutet.

**4.** Verwendung nach einem der vorhergehenden Ansprüche, dadurch gekennzeichnet, daß die Gruppe R in der Formel (I) eine gesättigte geradkettige Alkylgruppe mit 4 bis 11 Kohlenstoffatomen bedeutet.

**5.** Verwendung nach einem der vorhergehenden Ansprüche, dadurch gekennzeichnet, daß das Salicylsäurederivat unter 5-(n-Octanoyl)-salicylsäure, 5-(n-Decanoyl)-salicylsäure und 5-(n-Dodecanoyl)-salicylsäure ausgewählt ist.

**6.** Verwendung nach einem der vorhergehenden Ansprüche, dadurch gekennzeichnet, daß das Salicylsäurederivat 5-(n-Octanoyl)-salicylsäure ist.

**7.** Verwendung nach einem der vorhergehenden Ansprüche, dadurch gekennzeichnet, daß das Salicylsäurederivat in Form eines Salzes mit einer anorganischen oder organischen Base vorliegt.

**8.** Verwendung nach einem der vorhergehenden Ansprüche, dadurch gekennzeichnet, daß das Salicylsäurederivat oder sein Salz in einer Konzentration von 0,01 bis 2 Gew.-%, bezogen auf das Gesamtgewicht der Zusammensetzung, vorliegt.

**9.** Verwendung nach einem der vorhergehenden Ansprüche, dadurch gekennzeichnet, daß die Zusammensetzung ferner ein UV-Filter enthält.

**10.** Verwendung nach dem vorhergehenden Anspruch, dadurch gekennzeichnet, daß das UV-Filter unter Titanoxid oder Zinkoxid ausgewählt ist.

**11.** Verwendung nach einem der vorhergehenden Ansprüche, dadurch gekennzeichnet, daß die Zusammensetzung eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein öliges Gel, ein wasserfreies Gel, ein Serum oder eine Vesikeldispersion ist.

Activité Dopa-oxydase